# EUROPEAN PATENT APPLICATION

(11) **EP 1 728 509 A1**
(43) Date of publication of application: **06.12.2006**
(21) Application number: 05710716.1
(22) Date of filing: 25.02.2005
(51) Int. Cl.: A61K 31/352, A23L 1/30, A61P 1/02, C07D 311/62

(54) **PERIODONTAL LIGAMENT-PROTECTING AGENT**

(30) Priority: 26.03.2004 JP 2004091098
(71) Applicant: Asahi Breweries, Ltd., Tokyo 104-0031 (JP)
(72) Inventor: INABA, Hiroaki, Fukushima-ku, Osaka-shi Osaka 553-0004 (JP); TAGASHIRA, Motoyuki c/o Asahi Breweries, Ltd.;, Moriya-shi, Ibaraki 302-0106 (JP); KANDA, Tomomasa c/o Asahi Breweries, Ltd.;, Moriya-shi, Ibaraki 302-0106 (JP); AMANO, Atsuo, Suita-shi, Osaka 564-0061 (JP)
(74) Representative: Ziebig, Marlene
(86) International application number: PCT/JP2005/003166
(87) International publication number: WO 2005/092327

(57) **Abstract**

The present invention provides a protectant of periodontal membranes for inhibiting the destruction of periodontal tissues, particularly periodontal membranes by participation of *Porphyromonas gingivalis.* Further, it provides an agent for oral cavity or food and drinks containing the protectant of periodontal membranes having effects of prevention and therapy of diseases involving damage of periodontal membranes.

A protectant of periodontal membranes is proanthocyanidins, particularly proanthocyanidins derived from apple premature fruits or hop bracts having an effect decreasing the damage to periodontal membranes caused by *Porphyromonas gingivalis,* and an eating and drinking product containing the protectant of periodontal membranes as an effective component.

## Description

### Technical Field

The present invention relates to a protectant of periodontal membranes that is a proanthocyanidin-like polyphenol effective for decreasing damage of periodontal membranes derived from *Porphyromonas gingivalis,* particularly preferable polyphenol come from hop bracts or apple premature fruits, and the use.

### Background Art

As is commonly said, chew brings happy life. It is a doubtless fact that loss of teeth greatly reduces human QOL (quality of life).
Lately, the total amount of medical costs is about 30 trillion-yen and that of dental costs reaches 2.5 trillion-yen. Promotion of dental health is expected not only to reduce the dental costs but also to reduce the total medical costs. Namely, as a result of recent studies led by researchers in USA and other countries, there are many reports that the oral health, particularly in the absence of periodontal diseases, influences health of the whole body. In our countries, according to reports of the dental association in Hyogo prefecture ([the relation between "8020 campaign" and medical costs], the second research, 2002), a person having more teeth (a person having teeth of one's own) is healthy in conditions of the whole body and spend small sum of medical costs. It is considered that dental health will become important in aging society for the future.
Not only from a point of view of QOL, but also from a point of view of suppression and reduction of medical costs, development of a method for preventing the loss of teeth is industrially significant.

Lately, further, medical care for regenerating lost teeth and periodontal tissues is going to come into a practical stage in dental fields. As shown in the above, it is greatly significant to establish the technique.
Diseases of periodontal tissues, the so-called periodontal disease is an oral disease spread in the world and is one of main causes of teeth lost. It is almost established that the periodontal disease is an infective disease caused by bacteria. It may be caused by various bacteria in plaques in periodontal pockets. Particularly, it is considered that *Porphyromonas gingivalis* will be a main causal bacterium.
*P. gingivalis* is a bacterium detected in high frequency from a periodontal pocket of a patient and destroys periodontal tissues (periodontal membranes and alveolar bones) by production and release of a strong inflammatory protease (Arg-gingipain and Lys-gingipain). As the result, the periodontal tissues can not maintain and the teeth are lost.

The periodontal membranes mainly comprise of collagen fibrils. The one side of the fibrils is implanted in the alveolar bone and the other side is implanted in the cementum. The periodontal membranes act to bind teeth and alveolar bones, and are important tissues acting to absorb the press on the teeth and not to directly add the press to the bone. As described in the above, in the course developing the periodontal diseases, the destruction of periodontal membranes and alveolar bones is a particularly serious stage. Without the both tissues, teeth can not bear the force required in taking food.
As a trial of regeneration of teeth in dental fields, a method for regenerating lost periodontal membranes by using a growth factor of periodontal membrane cells and a method for culturing a sheet of periodontal membranes and grafting into a human oral cavity are studied. However, in such a case, when a patient is infected with *P. gingivalis,* the periodontal membranes of regenerating induction or graft are harmed by *P. gingivalis.* Accordingly, the membranes no protected cause troubles greatly.

As techniques for preventing and improving the periodontal diseases, it is known that plaques are removed by a physical method such as daily toothbrushing of individual persons, by polyphenol derived from green tea (Patent document 1), by polyphenol derived from trees (Patent document 2) or the like. The effect of these anti periodontal diseases is based on the inhibition effect of protease of *P*. *gingivalis,* or depression of affect and progress of periodontal diseases by inhibiting the adhesion of *P. gingivalis* to culture cells.
Patent document 1: Japanese Patent Laid-open Hei5-944 Publication
Patent document 2:Japanese Patent Laid-open Hei 8-81380 Publication

### Disclosure of Invention

### Problems to be dissolved by Invention

However, even if the adhesion of *P. gingivalis* to epithelial cells is inhibited, a small amount of *P*. *gingivalis* is adhered to form plaque, or even if the protease of *P. gingivalis* is efficiently inhibited, the effect is not directly confirmed. Because *P. gingivalis* secretes inflammatory factors such as LPS, it is difficult to evidence the inhibition of destruction of periodontal tissues that is an important stage aggravating the periodontal disease.
As to the protection effect of the periodontal tissues for regeneration in medical fields, there is no inventive technique.
The problems of the present invention are to provide a method for inhibiting the damage to periodontal membranes based on *P. gingivalis.*

### Disclosure of Invention

The inventors of the present invention have earnestly studied about the above problems, and they have found that a proanthocyanidin-like polyphenol is inhibiting the damage to periodontal membranes by *P. gingivalis* fungus and it has a protection effect. They have further confirmed that the polyphenol has an effect for decreasing the damage of *P*. *gingivalis* to the regeneration of induced cell membranes of the periodontal membranes. Then, they have completed the present invention by using the material as a protector of periodontal membranes in a medical drug, a quasi drug such as mouth wash liquid, or food and drinks. The word "polyphenol" means a compound having some phenolic hydroxyl groups in the molecular that is contained in plants. The word " proanthocyanidin-like polyphenol" means a compound producing anthocyanidin (cyanidin, delphinidin or pelargonidin) of a red color pigment by hydrolysis. Namely, the first invention is a protectant of periodontal membranes comprising a proanthocyanidin-like polyphenol. The second invention is a protectant of periodontal membranes comprising a proanthocyanidin-like polyphenol derived from unripe apples. The third invention is a protectant of periodontal membranes comprising a proanthocyanidin-like polyphenol derived from hop bracts.

As described in the above, one of the most important stages is destruction of periodontal membranes in the progress of periodontal diseases (aggravation of disease conditions). For resolving the problems of lost teeth based on the periodontal diseases, techniques for protecting the periodontal membranes is required. In the intraoral regeneration in medical fields, the formation of regenerated teeth is aimed. However, even if the regenerated teeth are adhered in a human oral cavity, the teeth can not be fixed when the tissues of periodontal membranes are lost. Then, the techniques for protecting the periodontal membranes are also required after the teeth have been lost. According to the present invention, the protectant can be used for strongly inhibiting the damage to periodontal membranes based on *P. gingivalis* fungus and used for a medical drug, a quasi drug such as mouth wash liquid, or food and drinks.

### Brief Description of Drawings

Fig. 1 shows the protection effect of periodontal membrane cells. The longitudinal axe shows the absorbance.
Fig. 2 shows the protection effect of periodontal membrane cells. The longitudinal axe shows the regenaration rate (%) of periodontal membranes. The holizontal axe shows the time (h).

### Best Mode for Embodying the Invention

The unripe apples as the raw materials for the present invention mean the superfluous apples artificially thinned out before ripening the fruits, or the apples naturally fallen from the apple trees.
The hop bracts as the raw materials for the present invention mean the bracts obtained by removing the lupulin gland part from hop cones. Generally, the hop cones are crushed and sieved, and the lupulin gland part is removed to obtain the hop bracts. In recent beer brewing, to save trouble for removing the bract parts, without removing the hop bracts useless in the beer brewing, the hop cones are formed into pellets for using in the beer brewing. As the raw materials in the present invention, there are no any problems when the hops are used without limiting to the hop cones containing the hop bracts or the hop pellets.

After the raw materials are compressed or extract with an aqueous alcohol solution, the obtained materials may be used as powder or liquid containing the materials for protecting periodontal membranes. If necessary, the materials are purified with a column packed resin beads having affinity for polyphenol, and the materials having high purity can be used.
The resulting materials for protecting periodontal membranes may be used for confectionery, food, drinks, most preferably, candy, chocolate, caramels, chewing gum and the like which are retained in the mouth for a long time. The materials further may be used by adding to oral agents such as gargle and dentifrice. When the materials are added into these food and drink or oral agents, the materials may be added as powder. Preferably, the materials are contained in an aqueous alcohol solution or alcohol liquid at a final concentration of 1-5000 ppm, preferably, 100-2000 ppm for the food and drink or oral agents.

### Example 1

### (Preparation of materials for protecting periodontal membranes from unripe apples)

Unripe apples (average weight 5.03g) 400g were homogenized with acidic methanol of 1% hydrochloric acid and the mixtures were extracted three times during heat refluxing. After the extract was concentrated in vacuo to remove methanol, chloroform was added, the mixture was partitioned, and the water phase was recovered and filtered to obtain 200ml with distilled water. After purifying the solution by a method for extracting the solid phase with Sec-pac C 18, the solution was freeze-dried to obtain the materials for protecting periodontal membranes 8.9g. The yield from the unripe apples was 2.2%.

### Example 2

### (Preparation of the materials for protecting periodontal membranes from hop bracts)

Hop bracts 50g was extracted with 1000ml of an aqueous solution of 40% ethanol by stirring at a temperature of 50°C for 60 minutes. After filtering, the extract was concentrated in vacuo to obtain a volume of 500ml. The concentrate was passed through a column filled with styrene-divinylbenzene resin (manufactured by Mitsubishi Chemical Corporation, Sepabeads SP 70) 150ml and the column was washed with 500ml of water. Then, 600ml of an aqueous solution of 50 % ethanol were passed through the column. The resulting solution was freeze-dried to obtain 1.7 g of the materials for protecting periodontal membranes as pale yellow powder having odorless weak bitter taste.
The yield from the hop bracts was 3.4%.

### Example 3

### (Further purification of the materials for protecting periodontal membranes with a ultrafilter)

Five grams of the materials for protecting periodontal membranes obtained by the same method as shown in Example 2 was dissolved in 500ml of an aqueous solution of 50% ethanol, and treated with a ultrafilter having 10,000 of fraction molecular weight. Upper remain liquid no passed the ultrafilter was concentrated and freeze-dried to obtain 2.2g of yellow-brown powder of further purified materials for protecting periodontal membranes.

### Example 4

### (Dentifrice)

| | |
|---|---|
| Dibasic calcium phosphate | 42.0 |
| Glycerin | 18.0 |
| Carrageenan | 0.7 |
| Sodium lauryl sulfate | 1.2 |
| Saccharin sodium | 0.09 |
| Butyl p-hydroxybenzoate | 0.005 |
| The materials obtained in Example 1 | 0.005 |
| Perfume | 1.0 |
| Water | 37.0 |
| Total | 100.0 |

Using the above constituents of each weight part, debtiflice was prepared by a usual method. Instead of the materials in Example 1, dentifrice was similarly prepared with the materials in Example 2 and Example 3. Example 5

### (Gargle)

| | |
|---|---|
| Glycerin | 7.0 |
| Sorbitol | 5.0 |
| Ethyl alcohol | 15.0 |
| Sodium lauryl sulfate | 0.8 |
| Saccharin sodium | 0.1 |
| 1-Menthol | 0.05 |
| Perfume | 0.045 |
| The materials obtained in Example 1 | 0.005 |
| Water | 72.0 |
| Total | 100.0 |

Using the above constituents of each weight part, gargle was prepared by a usual method. Instead of the materials in Example 1, gargle was similarly prepared with the materials in Example 2 and Example 3. Example 6

### (Troche)

| | |
|---|---|
| Gum Arabic | 6.0 |
| Magnesium stearate | 3.0 |
| Glucose | 73.0 |
| Lactose | 17.6 |
| Dipotassium hydrogen phosphate | 0.2 |
| Monobasic potassium phosphate | 0.1 |
| Perfume | 0.095 |
| The materials obtained in Example 1 | 0.005 |
| Total | 100.0 |

Using the above constituents of each weight part, troche was prepared by a usual method. Instead of the materials in Example 1, troche was similarly prepared with the materials in Example 2 and Example 3.

### Example 7

### (Wheat gluten)

| | |
|---|---|
| Sucrose | 20.0 |
| Millet jelly (75% solid) | 70.0 |
| Water | 9.5 |
| Coloring agent | 0.45 |
| Perfume | 0.045 |
| The materials obtained in Example 1 | 0.005 |
| Total | 100.0 |

Using the above constituents of each weight part, wheat gluten was prepared by a usual method. Instead of the materials in Example 1, wheat gluten was similarly prepared with the materials in Example 2 and Example 3. Example 8

### (Chewing gum)

| | |
|---|---|
| Gum base | 20.0 |
| Calcium carbonate | 2.0 |
| Lactose | 77.0 |
| Stevioside | 0.095 |
| The materials obtained in Example 1 | 0.005 |
| Perfume | 0.9 |
| Total | 100.0 |

Using the above constituents of each weight part, chewing gum was prepared by a usual method. Instead of the materials in Example 1, chewing gum was similarly prepared with the materials in Example 2 and Example 3. Example 9

### (Juice)

| | |
|---|---|
| Concentrated mandarin orange juice | 15.0 |
| Fructose | 5.0 |
| Citric acid | 0.2 |
| Perfume | 0.1 |
| Coloring agent | 0.15 |
| Sodium ascorbate | 0.048 |
| The materials obtained in Example 1 | 0.002 |
| Water | 79.5 |
| Total | 100.0 |

Using the above constituents of each weight part, juice was prepared by a usual method. Instead of the materials in Example 1, juice was similarly prepared with the materials in Example 2 and Example 3. Example 10

### (Cookies)

| | |
|---|---|
| Soft flour | 32.0 |
| Whole egg | 16.0 |
| Butter | 16.0 |
| Sugar | 25.0 |
| Water | 10.8 |
| Baking powder | 0.198 |
| The materials obtained in Example 1 | 0.002 |
| Total | 100.0 |

Using the above constituents of each weight part, cookies were prepared by a usual method. Instead of the materials in Example 1, cookies were similarly prepared with the materials in Example 2 and Example 3. Example 11

### (Caramels)

| | |
|---|---|
| Granulated sugar | 31.0 |
| Millet jelly (75% solid) | 20.0 |
| Powder milk | 40.0 |
| Hardened oil | 5.0 |
| Common salt | 0.6 |
| Perfume | 0.025 |
| The materials obtained in Example 1 | 0.005 |
| Water | 3.37 |
| Total | 100.0 |

Using the above constituents of each weight part, caramels were prepared by a usual method. Instead of the materials in Example 1, caramels were similarly prepared with the materials in Example 2 and Example 3. Example 12

### (Protecting effect of periodontal membrane cells)

Cells derived from periodontal membranes were used, after the cells were detached from the root center of the third molar teeth, subcultured at 5-7 passages in a DMEM culture medium, and the alkaline phosphatase activity was confirmed. In comparative example, epigallocatechin gallate derived from green tea was used as polyphenol. The materials for protecting periodontal membranes obtained in Examples 1, 2 and 3 were used. *P. gingivalis* fungi were ATCC3327 strains.
The cells of periodontal membranes of 1 × 10⁴ were inoculated on a plate of 96 wells. The cells were incubated at a temperature of 37°C for 12 hours to produce a single layer of the cells. After the culture medium was changed into a DMEM culture medium excluding FCS, the cells were incubated at a temperature of 37°C for 12 hours. Then, epigallocatechin gallate (Comparative example) and the materials of Example 1, 2 and 3 were added to the culture medium, *P. gingivalis* fungi of 100 times per the cell were added to the culture medium to infect the cells, the cells were incubated at a temperature of 37°C for 6 hours. After the incubation, the cells were washed with PBS three times, treated with an aqueous solution of 25% glutaraldehyde for 5 minutes and fixed. The fixed cells were further washed with PBS three times, and stained with a 20% methanol solution of 0.5% crystal violet. The cells were further washed with PBS five times, and the absorbance was measured with a platereader (BIO-Rad Co., type 680) at 590 nm.

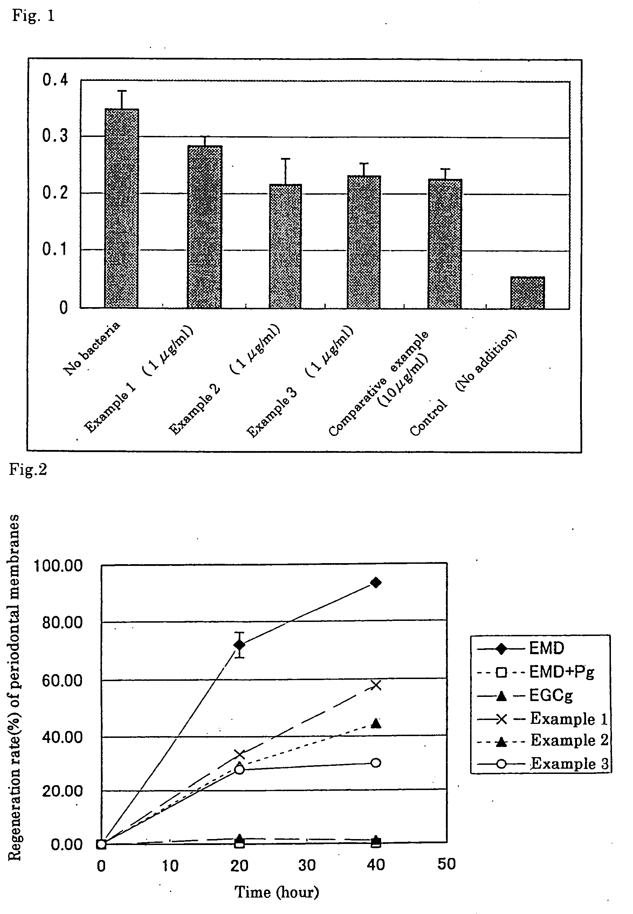

The crystal violet dyes the cells, the single layer of the cells are maintained and dyed well. Accordingly, the absorbance becomes high at 590 nm. On the other hand, when the damage of the cells becomes serious to destruct the single layer and few cells are dyed, the absorbance becomes low at 590 nm.
The test results are shown in Fig. 1. When the materials of Example 1, 2 and 3 were added (each 1 µg/ml), comparing with Comparative example (no polyphenol added), the damage of the periodontal membranes was low, and the membranes were preserved well. Accordingly high absorbance was shown. The effect was the same as that of 10 µg/ml of epigallocatechin gallate (Comparative example) or better. Namely, the effect of the materials of Example 1, 2 and 3 was the same or better than that of 10 times of the materials of Comparative example.

### Example 13

### (Protecting effect of periodontal membrane cells)

Cells derived from periodontal membranes were used, after the cells were detached from the root center of the third molar teeth, subcultured at 5-7 passages in a DMEM culture medium, and the alkaline phosphatase activity was confirmed. In comparative example, epigallocatechin gallate derived from green tea was used as polyphenol. The materials for protecting periodontal membranes obtained in Examples 1, 2 and 3 were used. *P. gingivalis* fungi were ATCC3327 strains. As a regeneration inducing agent of the periodontal membranes, emdogain (registered trademark, EMD) was used.
The cells of periodontal membranes of 1 × 10⁴ were inoculated on a plate of 96 wells that emdogain (registered trademark) was previously coated. The cells were incubated at a temperature of 37°C for 12 hours in a DMEM culture medium containing 10 % FCS to produce a single layer of the cells. After the culture medium was changed into a DMEM culture medium excluding FCS, the cells were incubated at a temperature of 37°C for 12 hours.
Then, the membranes were wound with a sharp pin, epigallocatechin gallate (Comparative example) and the materials of Example 1, 2 and 3 were added to the culture medium, *P. gingivalis* fungi of 100 times per the cell were added to the culture medium to infect the cells, and the cells were incubated at a temperature of 37°C for 40 hours. Before the incubation, after 20 and 40 hours of the incubation beginning, the wounded membranes were taken a photograph under a microscope. The regeneration conditions of the membranes were numerically indicated with an image treating apparatus.
The test results are shown in Fig. 2. When the materials of Example 1, 2 and 3 were added (each 10 µg/ml), comparing with Control (no polyphenol added: EMD + Pg) and 10 µg/ml of epigallocatechin gallate (Comparative example: EGCg), the regeneration of the periodontal membranes was apparently found. Industrial Applicability

The protectant of periodontal membranes of the present invention has a protection effect of the periodontal tissues for regeneration in medical fields. It can be used in a medical drug, a quasi drug such as mouth wash liquid, or food and drinks.

## Claims

1. A protectant of periodontal membranes comprising a proanthocyanidin-like polyphenol for inhibiting destruction of periodontal membranes by using *Porphyromonas gingivalis* fungus.

2. A protectant of periodontal membranes as claimed in claim 1, it is derived from hops, particularly preferable hop bracts.

3. A protectant of periodontal membranes as claimed in claim 1, it is derived from apples.

4. An agent for oral cavity containing the protectant of periodontal membranes as claimed in claim 1.

5. An agent for oral cavity containing the protectant of periodontal membranes as claimed in claim 2.

6. An agent for oral cavity containing the protectant of periodontal membranes as claimed in claim 3.

7. An eating and drinking product containing the protectant of periodontal membranes as claimed in claim 1.

8. An eating and drinking product containing the protectant of periodontal membranes as claimed in claim 2.

9. An eating and drinking product containing the protectant of periodontal membranes as claimed in claim 3.
